# EUROPEAN PATENT SPECIFICATION

(11) **EP 0 727 241 B1**
(45) Date of publication and mention of the grant of the patent: **17.09.2003**
(21) Application number: 96850031.4
(22) Date of filing: 13.02.1996
(51) Int. Cl.: A61N 1/36, A61B 5/04

(54) **Device for heart stimulation**
Vorrichtung zur Stimulation der Herzfunktion
Dispositif de stimulation cardiaque

(30) Priority: 20.02.1995 SE 9500620
(43) Date of publication of application: 21.08.1996
(73) Proprietor: St. Jude Medical AB, 175 84 Järfälla (SE)
(72) Inventor: Norén, Kjell, 171 58 Solna (SE); Hirschberg, Jakub, 183 44 Täby (SE)

(56) References cited:
- EP-A- 0 314 078
- PATENT ABSTRACTS OF JAPAN vol. 016, no. 369, 10 August 1992 (1992-08-10) & JP 04 117967 JP (NIHON SOUGOU IGAKU KENKIYUUSHIYO: YUUGEN), 17 April 1992 (1992-04-17)

## Description

The invention relates to a device for heart stimulation of the general type disclosed in the preamble to Claim 1.

Such devices for heart stimulation, specially as implants, but also those for external placement, are known in a wide range of variations. The most common is the pacemaker, which by controlled application of pulses can control the beating of the heart, either completely when the natural function is absent, or as a controlled supplement. In general, a pacemaker is often designed with a defibrillation function, i.e. for emitting powerful current shocks intended to break an unsuitable arythmic state (e.g. fibrillation). This is a rather brutal method of treatment and for many years there has been a desire to achieve gentler means of breaking off and preventing such states.

EP-A-0 314 078 describes a non-invasive electrical stimulator for pacing the heart. The stimulator generates a pulse having a ramped current waveform which rises gradually to the maximum amplitude. The rise time extends over at least one-half of the duration of the pulse. This should reduce muscle stimulation and pain associated with external stimulation of the heart.

In commonly occurring malfunctions of the heart muscles, instead of a consistent "ignition sequence" (to use a metaphor) propagating in a natural manner from the sinus node to produce a satisfactory heart beat, there is a type of cyclical process which does not dissipate after a full contraction but can continue as local contractions to no purpose. The conduction system has collapsed. A relatively mild therapy for correcting or restoring this system is desirable.

The main purpose of the invention is to achieve an electrical influence on the heart which in a gentle manner reduces the occurrence of malfunctions in the muscles of the heart.

Another purpose of the invention is to achieve an electrical effect on the heart which can be employed to stop at an early stage fibrillation and tachycardia.

An additional purpose of the invention is to achieve an electrical effect on the heart which can be used together with pace pulse stimulation and/or stimulation through defibrillation of the heart.

Another purpose of the invention is to achieve measurements of cardiac functions under special conditions for diagnostic purposes.

By applying a direct current field over the heart, it has been surprisingly shown in tests that it is possible to achieve a recovery of the correct conduction system in the heart muscles. In general, this application of dc current should be temporary and prompted by a demonstrated need.

It is also been demonstrated that the cardiac ECG will be markedly changed so that special disease states can be diagnosed by applying a dc field.

In certain cases there can, however, be a need for long term therapeutic application of a dc field. The dc field can then be applied over the electrodes. An electrode, which in this case acts as an anode, can then cause an undesirable stimulation of the tissue with which it is in contact. In such cases it is an advantage if the dc field changes polarity periodically. Thus in such cases an alternating current with a low frequency relative to the heart beat is applied to the heart.

The invention concerns a device for affecting the heart comprising heart affecting means controlled to apply a voltage signal of selectable polarity and duration between electrodes, said voltage signal having a duration from beginning to end of at least 30 ms. The heart affecting means are arranged to apply, during an activation interval, the voltage signal over at least a selected portion of the heart, the signal comprising voltage changes, such as rise and fall, with a derivative the absolute value of which is less than the absolute value of the derivative which for the patient in question causes a triggering of the patient's heartbeat , the absolute value of the derivative of the signal being less than 600 V/sec over the entire curve .

In accordance with the invention it is essential that the forward edge and the rearward edge of the voltage application increases or decreases so slowly that no heart beat is triggered. The falling off period should be just as long as the period of increase but in the opposite direction. The period of increase and the falling off period need not, however, be identical. Furthermore all signal transitions shall be gently rounded, also to avoid triggering a heart beat. The absolute value of the derivative of the signal is over the entire curve less than 600 volts/second. The duration of the applied voltage must be made long relative to the length of the pace pulser (ca 1 ms) or of the defibrillating pulse (<ca 10 ms) and is therefore at least 30 ms, is of course dependent on the individual in question and the duration can, however, extend over a number of heart beats and in certain cases over hours and days.

Without intending to restrict the invention to a certain explanation, it is assumed at present that the observed effect depends on the particular electrical/elektrochemical conditions in the active heart cells. The polarization in most heart cells at rest is ca -90 mV, with the inside negatively charged relative to the outside. An electric disturbance exceeding a certain threshold value causes a reversal of the cell polarity, a depolarisation. The amplitude of the action potential is about 110 mV and the voltage differential between the cell's interior and exterior is then +20 mV. Repolarization occurs spontaneously. The process is associated with the contraction of the muscle cell.

It is assumed that application of a constant voltage field over the heart muscles with a voltage differential between two electrodes, causes the absolute potential for the exterior of the cells in the potential field to be changed. A change in potential caused by the applied voltage due to the dc field may conceivably be insufficient to trigger the polarization.

In experiments on pigs, it has for example been shown that by applying an electric field (of 4 V) in the direction of the axis of the heart, there was obtained a phase shift between the atrium and ventricular depolarizations sufficient so that they will occur at the same time.

In accordance with a further development of the invention, a plurality of electrodes are arranged which can provide different types of fields by suitable application of potentials.

With four electrodes in a single plane, it is virtually possible to obtain for example a dipole field in any direction within this plane.

When working in conjunction with pace pulses, it is suitable to see to it that the field is applied when the pulse is applied, either by synchronization or by applying the field for so a long period that synchronization is not necessary. The application itself and its elimination must occur sufficiently slowly (long rising and falling times) so that it will not give rise to any depolarization effect.

The invention will be described in more detail below with reference to the accompanying drawings, where
Figure 1 shows a block diagram of one embodiment of a circuit in a heart stimulation device with the arrangement according to the invention,
Figure 2 shows various types of possible signal shapes which can be used according to the invention,
Figure 3 shows a simple block diagram of one embodiment of a circuit for carrying out therapy with voltage application according to the invention.

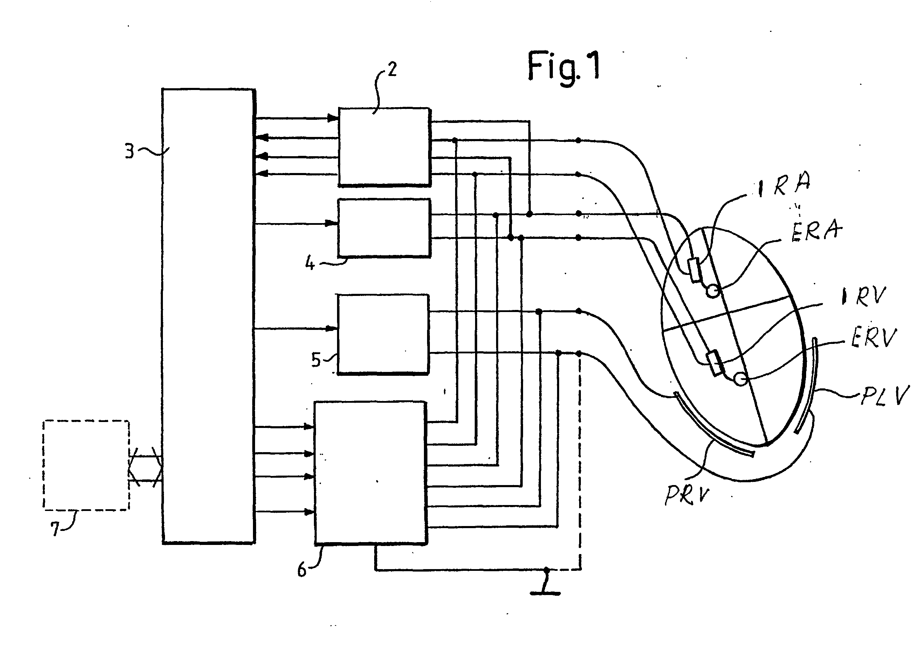

Figure 1 shows schematically a heart 1 in which a set consisting of a tip electrode ERA and a ring electrode IRA have been inserted in the upper right atrium and a set with a tip electrode ERV and a ring electrode IRV has been placed in the right ventricle. A first epicardial ventricle electrode PRV is placed outside the heart directly outside its right ventricle. A second epicardial ventricle electrode PLV is placed outside the heart directly outside its left ventricle.

This is a possible electrode configuration for a heart which is subject to "flutter" or "fibrillation". The electrode configuration is coupled to an implanted pacemaker/defibrillator.

Depending on the type of cardiac fault, the electrode set ERA, IRA or ERV, IRV can be eliminated (not shown in a special figure). The atrial functioning of the heart is measured between ERA/IRA and its ventricular function is measured between ERV/IRV. These signals are detected in a detection circuit 2 to which the electrodes are coupled. The detection circuit 2 compares the incoming signals with special predetermined conditions stored in the circuit, said conditions prompting electrical cardiac stimulations of various types. When one of these conditions is fulfilled, its signal is fed to a control circuit 3 that steps are to be taken. In Figure 1, several outputs are shown from the detection circuit 2 to the control circuit 3 to indicate that different conditions give different types of signals.

It should be observed that a heart stimulation device implanted in a patient's body should require as little energy as possible to be able to function for as long as possible without battery replacement, since such replacement requires reoperating on the patient. As in conventional pacing/pacemaker treatment, the instantaneous cardiac activity of the heart is measured for example by the electrodes ERA and/or ERV. Transmission between the circuits 2 and 3 and the calculations in the circuit 3 can of course be carried out both in an analogue or digital manner. The circuit designs themselves are known and do not constitute any portion of the invention. They are simple for a person skilled in the art to construct if he knows the desired function, and are therefore not shown here.

If the conditions for sending pace pulses to ERA and/or ERV are fulfilled, the control circuit 3 activates a pace pulse generating circuit 4 coupled to the electrodes ERA and ERV to provide one or more pace pulses. These are given between each electrode and a reference voltage V_{ref} with a displacement between them.

If the conditions for applying a defibrillation pulse or pulses are fulfilled, the control circuit 3 activates a defibrillator pulse generating circuit 5 coupled to PRV and PLV to provide some type of defibrillation pulse or defibrillation pulse sequence.

This belongs to the known art and will not be described in more detail here. The various circuits can operate and be controlled in a more or less complicated manner which will not be described in more detail here. It should, however, be observed that the various types and combinations of pace pulses, pace pulse sequences, defibrillation pulses and defibrillation pulse sequences can be utilized simultaneously with the therapy provided by the invention. It should also be observed that any of the abovementioned circuits except circuit 3, can be eliminated, for example circuit 4 and/or circuit 5 without departing from the scope of the invention.

According to the invention an additional circuit 6 is arranged and is controlled by the control circuit 3. The circuit 6 is shown in Figure 1 coupled to all of the electrodes outside and within the heart. This is due to the fact that for different states of the heart and/or for different conditions regarding the detected signals, it can be suitable to apply the long or low frequency non triggering stimulation signal according to the invention between various of the electrodes shown and/or between them and a reference potential V_{ref}, which is suitably on the casing of the implanted stimulating device according to the invention. It is also possible to provide a plurality of, for example 3, of the electrodes with simultaneous current and for example give a multi phase treatment.

It is, however, obvious that in individual cases there may only be required an applied slowly varying stimulation signal over only two of the electrodes, for example between the ventricural electrodes PRV and PLV, or between the ring electrodes IRA and IRV. In such a case the connection to the other electrodes from the circuit 6 are not required. Other electrodes can then be eliminated, if they are not required for another type of treatment.

As stated above, the voltage application according to the invention can assume various forms. It can consist of a single voltage application during a certain time period with a certain polarity, or it can consist of a slowly varying alternating current, or it can consist of a voltage application during a certain time period with one polarity followed by a period without extra voltage application and thereafter followed by a voltage application with the opposite polarity or alternatively with the same polarity, etc.

Measuring cardiac activity can be effected during voltage application externally or internally with the detection circuit 2 for diagnosis of the heart. It can be done for example during a medical check-up. An ECG (electrocardiogram) is a sequence of signals corresponding to various electrical events. These can be registered with the aid of electrodes on the surface of the body or with electrodes in the heart, as is shown in Figure 1. The relevant signal portions during a heart beat are identified with the letters P, RS (or merely R), T, where P corresponds to atrial polarization, QRS to ventricular polarization and T ventricular repolarization. An ECG is a diagnostically useful aid for studying, for example, damage to the heart muscle and its conduction system.

When applying low voltage direct current over portions of the heart, there is a distortion of the ECG, for example the above mentioned phase shift between the atrial and venticular depolarization. This distortion will be different for different cardiac pathological states and for different types of voltage application. An ECg taken during a slowly applied voltage over the heart, possibly with varying electrode configuration according to a predetermined pattern, can thus provide essential and detailed information to the examining physician concerning the state of the patient's heart. The arrangement according to the invention can therefore be used both for diagnosis and therapy.

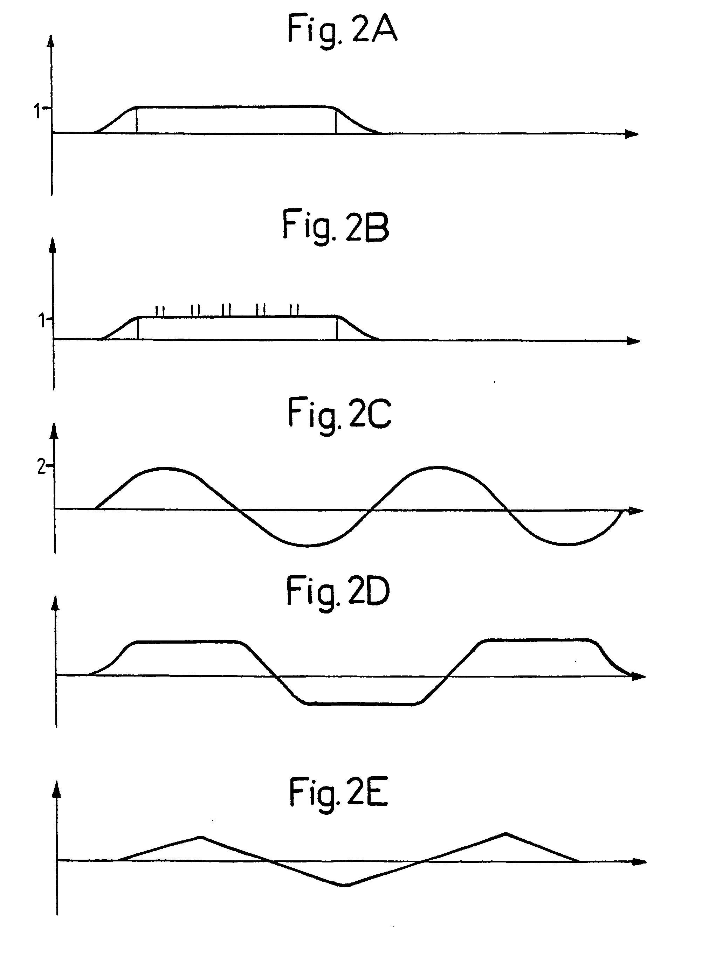

Figures 2A-2E show various curve shapes of the voltage application according to the invention. Figure 2A shows a voltage application during a certain number of heart beats with a soft start, a period of increase of approximately 400 V/sec up to approximately 2 V with a soft transition to constant voltage and a gently falling curve down to 0 V. Figure 2B shows the same curve as in Figure 2A but with superimposed pacing pulses. Figure 2C shows an applied sinus wave with a frequency which, for example, can be 1 Hz, and with an amplitude of approximately 2 V. Figure 2D shows a voltage application with alternating pulses with the pulse shape shown in Figures 2A and 2B. Fig. 2E shows that the voltage application can also have triangular shape. The pace pulse stimulation and the defibrillation stimulation can occur at the same time with each type of curve shape for voltage application according to the invention. Today, different voltage amplitudes are used depending on the method of cardiac stimulation:
- implantable pervenous and epicardial systems, 0 - 10 V,
- transcutaneous and osophagal diagnostic and ambulatory heart stimulations, 0-20 V.

It is essential that the frequency of the voltage application be sufficiently low to not affect the triggering of the cells. It should therefore exceed ten times the maximum conceivable pace pulse duration, which approximately corresponds to a duration of a half period of 30 ms, which means that the duration from beginning to the end of each applied voltage signal will probably be at least 30 ms. The absolute value of the derivative of each portion of the applied therapy voltage should be less than 600 V/sec in order not to cause a triggering of the heart. The amplitu-de of the applied voltage should be as low as possible while still achieving the required effect (a physiologically acceptable voltage is assumed). This involves an amplitude of from ca 60 mV and upwards to approximately 10 V, at the same time as the current can be between 0 and 10 mA.

What probably happens during the application of the voltage, at least when it is positive, is that normal heart cells are caused to act as sinus cells, which produces active stimulation. The heart can for example be caused to beat more rapidly thereby. A suitable level for the individual heart is best determined by analyzing the signals received from the measuring electrode or measuring electrodes ERA, ERV.

An advantage with the therapy which can be provided with the stimulation unit according to the invention is that it can be employed at a significantly earlier stage, when the heart comes into an arythmic state, than what is suitable for starting defibrillation. The conditions for defibrillation are relatively strict. The stimulation according to the invention can thus be employed even upon moderate ventricular and atrial arythmias.

In order to determine the suitable voltage level for the individual, in which the stimulation devices implanted is possible at a first occurrence of arythmia with the less strict conditions for employment to cause the applied voltage to increase very slowly.

Determination of when the arythmia declines or ceases can be done continually during a certain time period with increasing voltage. Thereafter, the voltage can be kept constant during a certain period of time to thereafter slowly decline. The voltage where the therapy had an effect can be stored in a memory 7 to be used at later occurrences of arythmia. To produce the suitable voltage level, a flank can be standardized and steeper than during the above described determination phase, but less steep than the flank which would trigger the patient's heart.

At later occurrences of arythmia, if the detection of the circuit 2 during treatment should show poor effect on the cardiac arythmia, there will be a new increase in the voltage level until a good effect is obtained and this will be stored in the memory 7. The applied voltage should, however, not be too great.

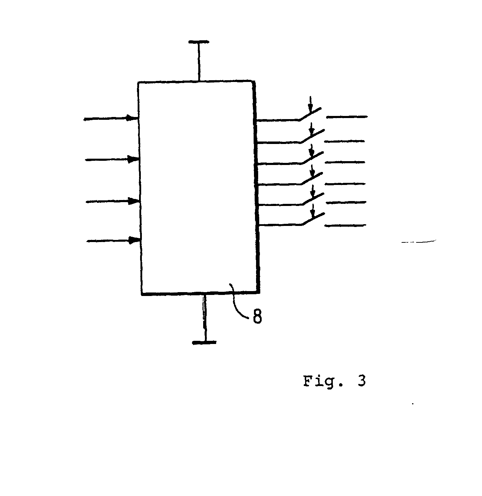

As shown in Figure 3, one embodiment of the circuit 6 can comprise an oscillator 8, which can be controlled by the control circuit 3 as regards its frequency, amplitude and curve shape. The control circuit 3 can also turn it on and off. The control circuit 3 can also control the engagement of the oscillator 8 to various combinations of the electrodes placed in and outside the heart. The detector circuit 2 also sends information to the control circuit concerning the timing of the heart beat. The control circuit 3 waits until after a fully completed heart beat with a dissipated so- called T-pulse before initiating an activation interval according to the invention.

A special effect can be achieved by controlling, in a cyclical sequence, during a first interval, the voltage application between the ventricular electrodes PRV and PLV, during a second interval between the tip electrodes ERA and ERV and during a third interval between the ring electrodes IRA and IRV and, for example, carrying out this control at positive voltage. Thus the effect of the anode on the adjacent tissue will not be a problem during long term therapy with voltage application, in an analogous manner to the application of low frequency voltage.

A plurality of electrodes can also be controlled at the same time to provide a multiphase effect, for example. In this case the oscillator 8 comprises several separate oscillators for example, which can be individually controllable as regards phase and preferably also as regards frequency and amplitude from the control circuit 3. The oscillator 8 can also be controlled to provide a varying output signal with the same sign, either in the form of half period or as variation amplitudes about a direct voltage.

The very slow increase in voltage when determining the suitable voltage can be achieved for example by controlling the oscillator to a very low frequency. It is also possible for this purpose to have an extra engageable ramp circuit with a long rise period and with a gentle transition to a sloping decline period (not shown).

## Claims

1. Device for affecting the heart comprising heart affecting means (3,4,5,6) controlled to apply a voltage signal of selectable polarity and duration between electrodes, said voltage signal having a duration from beginning to end of at least 30 ms, the heart affecting means (3,4,5,6) being arranged to apply, during an activation interval, the voltage signal over at least a selected portion of the heart, the signal comprising voltage changes, such as rise and fall, with a derivative the absolute value of which is less than the absolute value of the derivative which for the patient in question causes a triggering of the patient's heartbeat, the absolute value of the derivative of the signal being less than 600V/sec over the entire curve.

2. Device according to Claim 1, which is also provided with sensor means (2) for determining anomalistic states and for emitting an anomaly signal, **characterized by** a control circuit (3) which, in response to the anomaly signal, emits a start signal to the heart affecting means to activate the same for at least one activation interval of said type.

3. Device according to one of the preceding claims, **characterized in that** the heart affecting means comprises an oscillator (8), which emits a low frequency alternating voltage with the frequency which is less than 16 Hz, each half period of the alternating voltage constituting an activation interval.

4. Device according to Claim 2 or 3, **characterized in that** the heart affecting means is controllable as regards the length of period and/or amplitude and/or phase from the control circuit (3), either through external control or in response to a sensed state.

5. Device according to one of the preceding claims, **characterized in that** it cooperates superimposed with a pace pulse stimulating (4) and/or defibrillation stimulating (5) arrangement for a heart.

6. Device according to one of the preceding claims, **characterized in that** the heart affecting means for the controlled application of voltage during the application interval emits a sinusoidal signal.

7. Device according to one of Claims 1-5, **characterized in that** the heart affecting means for the controlled application of voltage during the activation interval or activation intervals emits a trapezoidal signal with gentle transitions of the derivative between different portions of the signal.

8. Device according to one of Claims 1-5, **characterized in that** heart affecting means for the controlled application of voltage during activation intervals emits a triangular signal with a gentle transition of the derivative between the rising and the falling portion.

9. Device according to one of the preceding claims, **characterized in that** the amplitude of the heart affecting means for the controlled application of voltage during activation intervals is controllable by the control device (3) between 0 and 10 V.

10. Device according to one of the preceding claims, **characterized in that** the heart affecting means during the activation interval or activation intervals provide the controlled application of voltage either via an attached left or right ventricular electrode (PRV, PLV) placed on the exterior of the heart, an electrode (IRA, ERA) in the atrium and an electrode (IRV, ERV) in the ventricle or an electrode in either the atrium or the ventricle and a reference electrode placed outside the heart, or a combination of these elec-trodes controllable from the control device (3) for connection to the heart affecting means in a cyclical sequence.

11. Device according to one of the preceding claims, **characterized in that** the heart affecting means during activation intervals provides the controlled application of voltage simultaneously via more attached electrodes than two.

12. Device according to one of the preceding claims, **characterized in that** each electrode of heart affecting means, coupled to the device is assigned a defined voltage so that each pair of attached electrodes together with the heart stimulation arrangement constitutes a source of cardiac therapy.

13. Device according to one of Claims 1-9, **characterized in that** the heart affecting means is placed on the exterior of a patient's body.

## Patentansprüche

1. Eine Vorrichtung zur Beeinflussung des Herzens, die ein Herzbeeinflussungsmittel (3, 4, 5, 6) umfasst, das so gesteuert wird, dass es ein Spannungssignal mit einer wählbaren Polarität und Dauer zwischen Elektroden anlegt, wobei das Spannungssignal eine Dauer vom Beginn zum Ende von mindestens 30 ms hat, wobei das Herzbeeinflussungsmittel (3, 4, 5, 6) so angeordnet ist, dass es während eines Aktivierungsintervalls das Spannungssignal über mindestens einen ausgewählten Abschnitt des Herzens anlegt, wobei das Signal Spannungsänderungen wie einen Anstieg und einen Abfall umfasst, wobei der Absolutbetrag einer Ableitung geringer ist als der Absolutbetrag der Ableitung, die bei dem betroffenen Patienten ein Auslösen des Herzschlags verursacht, wobei der Absolutwert der Ableitung des Signals über die gesamte Kurve geringer als 600 V/s ist.

2. Vorrichtung nach Anspruch 1, die auch mit einem Sensormittel (2) zur Bestimmung anomaler Zustände und zum Abgeben eines Anomaliesignals ausgestattet ist, **gekennzeichnet durch** eine Steuerschaltung (3), die als Reaktion auf das Anomaliesignal an das Herzbeeinflussungsmittel ein Startsignal zur Aktivierung des Herzbeeinflussungsmittels für mindestens ein Aktivierungsintervall der genannten Art abgibt.

3. Vorrichtung nach einem der vorstehenden Ansprüche, **dadurch gekennzeichnet, dass** das Herzbeeinflussungsmittel einen Oszillator (8) umfasst, der eine niederfrequente Wechselspannung mit einer Frequenz von weniger als 16 Hz abgibt, wobei jede Halbperiode der Wechselspannung ein Aktivierungsintervall darstellt.

4. Vorrichtung nach Anspruch 2 oder 3, **dadurch gekennzeichnet, dass** das Herzbeeinflussungsmittel bezüglich der Länge der Periode und/oder der Amplitude und/oder der Phase von der Steuerschaltung (3) steuerbar ist, und zwar entweder durch eine externe Steuerung oder als Reaktion auf einen abgefühlten Zustand.

5. Vorrichtung nach einem der vorstehenden Ansprüche, **dadurch gekennzeichnet, dass** sie überlagert mit einer Schrittmacherimpuls-Stimulationsanordnung (4) und/oder einer Defibrillations-Stimulationsanordnung (5) für ein Herz zusammenwirkt.

6. Vorrichtung nach einem der vorstehenden Ansprüche, **dadurch gekennzeichnet, dass** das Herzbeeinflussungsmittel zum gesteuerten Anlegen einer Spannung während des Anlegeintervalls ein sinusförmiges Signal abgibt.

7. Vorrichtung nach einem der Ansprüche 1 bis 5, **dadurch gekennzeichnet, dass** das Herzbeeinflussungsmittel zum gesteuerten Anlegen einer Spannung während des Aktivierungsintervalls oder der Aktivierungsintervalle ein trapezförmiges Signal mit sanften Übergängen der Ableitung zwischen verschiedenen Abschnitten des Signals abgibt.

8. Vorrichtung nach einem der Ansprüche 1 bis 5, **dadurch gekennzeichnet, dass** das Herzbeeinflussungsmittel zum gesteuerten Anlegen einer Spannung während der Aktivierungsintervalle ein dreieckförmiges Signal mit sanften Übergängen der Ableitung zwischen dem ansteigenden und dem abfallenden Abschnitt abgibt.

9. Vorrichtung nach einem der vorstehenden Ansprüche, **dadurch gekennzeichnet, dass** die Amplitude des Herzbeeinflussungsmittels zum gesteuerten Anlegen einer Spannung während der Aktivierungsintervalle durch die Steuervorrichtung (3) zwischen 0 und 10 V steuerbar ist.

10. Vorrichtung nach einem der vorstehenden Ansprüche, **dadurch gekennzeichnet, dass** das Herzbeeinflussungsmittel während des Aktivierungsintervalls oder der Aktivierungsintervalle das gesteuerte Anlegen einer Spannung entweder über eine angebrachte links- oder rechtsventrikuläre Elektrode (PRV, PLV), die außen am Herzen platziert ist, eine Elektrode (IRA, ERA) im Atrium und eine Elektrode (IRV, ERV) im Ventrikel oder eine Elektrode entweder im Atrium oder im Ventrikel und eine Bezugselektrode, die außerhalb des Herzens platziert ist, oder über eine Kombination dieser Elektroden bereitstellt, die von der Steuervorrichtung (3) zur Verbindung mit dem Herzbeeinflussungsmittel in einer zyklischen Sequenz steuerbar sind.

11. Vorrichtung nach einem der vorstehenden Ansprüche, **dadurch gekennzeichnet, dass** das Herzbeeinflussungsmittel während der Aktivierungsintervalle das gesteuerte Anlegen einer Spannung gleichzeitig über mehr als zwei angebrachte Elektroden bereitstellt.

12. Vorrichtung nach einem der vorstehenden Ansprüche, **dadurch gekennzeichnet, dass** jeder Elektrode des Herzbeeinflussungsmittels, die mit der Vorrichtung gekoppelt ist, eine bestimmte Spannung zugewiesen wird, so dass jedes Paar von angebrachten Elektroden zusammen mit der Herzstimulatiorisanordnung eine Quelle für die Herztherapie bildet.

13. Vorrichtung nach einem der Ansprüche 1 bis 9, **dadurch gekennzeichnet, dass** das Herzbeeinflussungsmittel außen am Körper eines Patienten platziert wird.

## Revendications

1. Dispositif pour affecter le coeur, comportant des moyens (3, 4, 5, 6) d'affectation du coeur commandés de manière à appliquer un signal de tension de polarité pouvant être sélectionné et de durée pouvant être sélectionnée entre des électrodes, le signal de tension ayant une durée allant du début à la fin d'au moins 30 ms, les moyens (3, 4, 5, 6) d'affectation du coeur étant agencés de manière à appliquer, pendant un intervalle d'activation, le signal de tension sur au moins une partie sélectionnée du coeur, le signal comportant des variations de tension, telles que des montées et des chutes, dont la dérivée de la valeur absolue est moindre que la valeur absolue de la dérivée qui, pour le patient en question, entraîne un déclenchement du battement cardiaque du patient, la valeur absolue de la dérivée du signal étant inférieure à 600 V/sec sur toute la courbe.

2. Dispositif suivant la revendication 1, qui est également muni de moyens (2) formant capteur pour déterminer des états anormales et pour émettre un signal d'anomalie, **caractérisé par** un circuit (3) de commande qui, en réponse au signal d'anomalie, émet un signal de départ pour les moyens d'affectation de coeur pour activer ces derniers pour au moins un intervalle d'activation dudit type en question.

3. Dispositif suivant l'une des revendications précédentes, **caractérisé en ce que** les moyens d'affectation comportent un oscillateur (8), qui émet une tension alternative à basse fréquence, dont la fréquence est moindre que 16 Hz, chaque demi-période de la tension alternative constituant un intervalle d'activation.

4. Dispositif suivant la revendication 2 ou 3, **caractérisé en ce que** les moyens d'affectation du coeur peuvent être commandés en ce qui concerne la longueur de la période et/ou l'amplitude et/ou la phase du circuit (3) de commande, soit par une commande externe, soit en réponse à un état détecté.

5. Dispositif suivant l'une des revendications précédentes, **caractérisé en ce qu'**il coopère, en étant superposé avec un agencement (4) de stimulation d'impulsions et/ou un agencement (5) de stimulation par défibrillation pour un coeur.

6. Dispositif suivant l'une des revendications précédentes, **caractérisé en ce que** les moyens d'affectation du coeur pour l'application commandée de tension pendant l'intervalle d'application émet un signal sinusoïdal.

7. Dispositif suivant l'une des revendications 1 à 5, **caractérisé en ce que** les moyens d'affectation du coeur pour l'application commandée de tension pendant l'intervalle d'activation ou des intervalles d'activation émet un signal trapézoïdal avec des transitions en douceur de la dérivée entre différentes parties du signal.

8. Dispositif suivant l'une des revendications 1 à 5, **caractérisé en ce que** des moyens d'affectation du coeur pour l'application commandée de tension pendant des intervalles d'activation émet un signal triangulaire avec une transition en douceur de la dérivée entre la partie croissante et la partie décroissante.

9. Dispositif suivant l'une des revendications précédentes, **caractérisé en ce que** l'amplitude des moyens d'affectation du coeur pour l'application commandée de tension pendant des intervalles d'activation peut être commandée par le dispositif (3) de commande entre 0 et 10 V.

10. Dispositif suivant l'une des revendications précédentes, **caractérisé en ce que** les moyens d'affectation du coeur pendant l'intervalle d'activation ou les intervalles d'activation fournissent l'application commandée de tension, soit par l'intermédiaire d'une électrode ventriculaire gauche ou droite (PRV, PLV), placée à l'extérieur du coeur, une électrode (IRA, ERA) dans l'oreillette et une électrode (IRV, ERV) dans le ventricule ou une électrode soit dans l'oreillette, soit dans le ventricule et une électrode de référence placée à l'extérieur du coeur, ou une combinaison de ces électrodes pouvant être commandées par le dispositif (3) de commande pour une connexion aux moyens d'affectation du coeur suivant une séquence cyclique.

11. Dispositif suivant l'une des revendications précédentes, **caractérisé en ce que** les moyens d'affectation du coeur pendant des intervalles d'activation fournissent l'application commandée de tension simultanément par l'intermédiaire de plus d'électrodes fixées que deux.

12. Dispositif suivant l'une des revendications précédentes, **caractérisé en ce qu'**à chaque électrode des moyens d'affectation du coeur, couplée au dispositif, il est affecté une tension définie, de sorte que chaque paire d'électrode fixée ensemble avec l'agencement de stimulation cardiaque constitue une source de thérapie cardiaque.

13. Dispositif suivant l'une des revendications 1 à 9, **caractérisé en ce que** les moyens d'affectation du coeur sont placés à l'extérieur du corps d'un patient.
